Europäisches Patentamt

European Patent Office

Office européen des brevets

(11) Publication number 0 013 459
A2

# EUROPEAN PATENT APPLICATION

(21) Application number: 79200804.7

(22) Date of filing: 24.12.79

(51) Int. Cl.³: **A 61 K 7/48**
**A 61 K 9/10**

(30) Priority: 08.01.79 US 1974

(43) Date of publication of application:
23.07.80 Bulletin 80/15

(84) Designated Contracting States:
BE DE FR GB IT NL

(71) Applicant: THE PROCTER & GAMBLE COMPANY
301 East Sixth Street
Cincinnati Ohio 45202(US)

(72) Inventor: Wickett, Richard Randall
4034 Jennifer Dr.
Hamilton, Ohio 45013(US)

(72) Inventor: Cooper, Eugene Rex
2424 Ambassador Dr.
Cincinnati, Ohio 45231(US)

(72) Inventor: Loomans, Maurice Edward
5231 Jessup Rd.
Cincinnati, Ohio 45239(US)

(74) Representative: Munro, Hamish David et al,
PROCTER & GAMBLE EUROPEAN TECHNICAL CENTER
Temselaan 100
B-1820 Strombeek-Bever(BE)

(54) Topical anti-acne compositions.

(57) Compositions employing benzoyl peroxide together with a penetrating carrier comprising a $C_6$-$C_{14}$ primary alkanol and a propane- or butane-diol are disclosed for the treatment of acne and similar skin lesions by topical application.

EP 0 013 459 A2

0013459

TOPICAL ANTI-ACNE COMPOSITIONS

This invention relates to topical compositions comprising benzoyl peroxide and certain topical carriers as well as processes for the topical use of such compositions. More particularly, the invention relates to topical compositions for the treatment of acne and similar skin lesions and methods for treating acne whereby benzoyl peroxide is delivered through skin and other barrier tissue to the underlying focus of infection.

Acne and seborrhea are conditions of the human skin characterized by an excessive flow of sebum, or skin oil, from the sebaceous glands located in the pilosebaceous apparatus. Sebum reaches the skin surface through the ducts of the sebaceous follicles. One of the early events leading to acne lesions is hyperkeratinization. Hyperkeratinization leads to the formation of microcomedones which obstruct the sebaceous follicle. This obstruction leads to the clinical manifestations of acne such as papules, pustules, open comedones, or cysts. The environment of the microcomedo is conducive for the growth of bacteria which cause some aspects of the inflammatory response. Acne is particularly characterized by the presence of comedones, inflammatory papules, pustules, or cysts. The effect of acne ranges from slight skin irritation and pitting to disfiguring scars.

Many topical therapeutic agents are employed in the treatment of acne and seborrhea to prevent the blocking of the follicular duct, or, once it has become blocked, to act against the bacteria, and to provide combinations of each of these actions. The horny outer layer of the skin (stratum corneum) is formed of dead cells composed largely of keratin. Therapeutic agents which act to prevent the blocking of the follicular duct by promoting the removal or sluffing off of excess keratin are known as keratolytic agents. Sulfur, resorcinol and salicylic acid have been used as keratolytic agents in the treatment of acne.

For over sixty years, benzoyl peroxide has been used as a keratolytic agent in the topical treatment of skin lesions such as burns, varicose ulcers, sycosis vulgaris, seborrhea, and acne. Benzoyl peroxide, $(C_6H_5CO)_2O_2$, is a colorless, odorless, tasteless, crystalline solid, stable at ordinary room temperatures; it is a powerful oxidizing agent, yet generally safe for human use. As noted, benzoyl peroxide has been used as a very effective topical keratolytic and antibacterial agent in the treatment of acne. While benzoyl peroxide is an effective topical agent for the treatment of acne in formulations currently available, there is a continuing search for more effective delivery vehicles.

It has now been discovered that certain penetrating aids cause benzoyl peroxide to penetrate through keratinized structures such as the stratum corneum. It is believed that the increased keratolytic and antibacterial benefits of the compositions of the present invention result from the increased penetration of benzoyl peroxide into the keratinized structures which form the plugs in the sebaceous follicles.

When used in the treatment of acne, benzoyl peroxide produces dryness, exfoliation, and a decrease in bacterial flora. The use of benzoyl peroxide in topical compositions for treating skin lesions such as burns, varicose ulcers, sycosis vulgaris, and acne has been known for some 60 years. Levine, et al., Ohio State Med. J. 65, 492 (1969).

Benzoyl peroxide has been used in various formulations containing standard cosmetic ingredients. Broad categories of emollients and humectants have been disclosed for use with benzoyl peroxide. For example, U.S. Patent 4,075,353, Mandy, et al., February 21, 1978 describes a process for treating acarid skin infections of animals by topically applying benzoyl peroxide. The compositions disclosed include benzoyl peroxide and "nonreactive carrier materials" such as $C_2$ to $C_{20}$ aliphatic alcohols, glycols including ethylene glycol and propylene glycol, and $C_{12}$-$C_{22}$ fatty acids and their esters.

U.S. Patent 4,052,515, MacDermott, et al., October 4, 1977 claims a method of treating acne by applying a composition containing a $C_6$-$C_{12}$ unsaturated or saturated alcohol and a $C_2$-$C_3$ monoalcohol; propylene glycol is disclosed as a diluent.

An additional patent relates to antiacne preparations. U.S. Patent 3,535,422, Cox, et al., October 20, 1970 relates to stable benzoyl peroxide compositions containing organic emollients. The compositions include emollients selected from $C_4$-$C_{20}$ aliphatic alcohols, $C_2$-$C_3$ glycols, $C_{12}$-$C_{20}$ fatty acids and their esters, and mixtures thereof.

Although the workers in the art have made broad disclosures of preparations using benzoyl peroxide, they do not appear to have realized that the specific ingredients and compositional ranges of use taught herein provide unique, skin penetration enhancing benefits.

- 4 -

## DESCRIPTION OF THE INVENTION

The present invention encompasses compositions for use in the topical treatment of acne and similar skin lesions comprising from about 0.5% to about 25% benzoyl peroxide; and from about 8% to about 99% of a penetrating carrier consisting essentially of a $C_6$-$C_{14}$ primary alkanol, or mixtures thereof, and a diol selected from 1,2-propanediol, 1,2-butanediol, 1,3-butanediol, and 2,3-butanediol, or mixtures thereof, the weight ratio of primary alcohol to diol being in the range from about 5:1 to about 1:30; the balance of the composition comprising cosmetically acceptable excipients.

The present invention also encompases a method for treating acne and similar skin lesions, comprising applying to the afflicted situs a safe and effective amount of a composition comprising: (a) from about 0.5% to about 25% by weight of benzoyl peroxide; (b) from about 1% to about 40% of a $C_6$-$C_{14}$ primary alkanol, or mixtures thereof; (c) from about 5% to about 85% by weight of a diol selected from 1,2-propanediol, 1,2-butanediol, 1,3-butanediol, and 2,3-butanediol, or mixtures thereof; and (d) the balance, water and optional excipients.

It has now been discovered that benzoyl peroxide formulations containing the ingredients described above increase the penetration of benzoyl peroxide across the stratum corneum, and, it is believed, into the follicular keratinous tissue (especially comedones) that leads to clinically observable acne. The increased penetration results in increased keratolytic and antibacterial benefits, and provides more effective control of the symptoms of acne.

Benzoyl peroxide is an item of commerce. The compositions of the present invention comprise from about 0.5% to about 25% benzoyl peroxide, preferably from about 1% to about 15%, and most preferably from about 2% to about 10% benzoyl peroxide.

By "penetrating carrier" as used herein is meant the skin penetration promoting mixture of primary alcohol and diol used with benzoyl peroxide. The topical penetrating carrier is typically in the form of a cream, gel, or lotion. The topical carrier consists essentially of two critical ingredients, a $C_6$ to $C_{14}$ primary alcohol and a diol in a weight ratio of from about 5:1 to about 1:30, preferably from about 1:1 to about 1:15 and most preferably from about 1:2 to about 1:10.

The anti-acne compositions herein typically contain from about 1% to about 40% of the $C_6$ to $C_{14}$ primary alcohol, or mixtures thereof. Exemplary n-alcohols are hexanol octanol, nonanol and decanol; lauryl or myristyl alcohol are preferred, and myristyl alcohol is most preferred. The $C_6$ to $C_{14}$ primary alcohol is preferably present in the composition at from about 1% to about 10% by weight, and is most preferably present at about 2% to about 5% by weight.

The anti-acne compositions herein also typically contain from about 5% to about 85% by weight of a diol selected from 1,2-propanediol, 1,2-butanediol, 1,3-butanediol, 2,3-butanediol, or mixtures thereof; 1,2-propanediol is preferred. (Ethylene glycol and 1,3-propanediol do not aid the skin penetration of benzoyl peroxide.) The diol component is preferably present at from about 7% to about 35% by weight, and most preferably from about 9% to about 15% by weight.

The compositions typically comprise cosmetic excipients such as water and water/ethanol mixtures. Additionally, chelating agents such as EDTA, nitrilotriacetate, and gluconic, citric, and tartaric acids, preferably at a level of from 0.01% to 1% of the composition, can be used to avoid the decomposition of the peroxide compound by metal ions.

Other optional components such as finely divided sulfur, USP grade, at a level of from 1% to 25% of the composition, thickening agents, such as methyl cellulose, cross-linked carboxyl polymethylene polymers, bentonite,

gum tragacanth, gum acaraya, and polyethylene glycols, at a level of from 1% to 10% of the composition, and trace amounts of fragrance materials such as perfumes can also be used. Cosmetic resins and film formers can also be present.

By the term "comprising" herein is meant that various other, compatible ingredients can be present in the compositions as long as benzoyl peroxide and the critical components of the topical carrier, n-alcohol and diol, are present. The term comprising thus encompasses the more restrictive terms "consisting essentially of" and "consisting of".

All percentages herein are weight/weight.

The compositions are typically prepared by thoroughly blending all of the components together in admixture, and milling, if necessary, to reduce all particles of benzoyl peroxide to impalpable size (typically less than 0.25 mm). The benzoyl peroxide should be of high purity, on the order of 97% to 100% pure, and in the form of a finely divided powder. The powder may be either wet or dry, but is preferably wet for ease of handling and safety. If wet benzoyl peroxide is used, it may be necessary to grind the crystals before admixture or to mill the composition after admixture and blending to reduce the crystals to impalpable size. Preferably, the milling or grinding operation is performed in the cold to prevent decomposition of the peroxide by localized friction.

The compositions are typically applied twice daily to acne lesions. A typical safe and effective usage rate is about 0.001 $g/cm^2$ skin to about 0.5 $g/cm^2$ skin, per application, but this can vary with the user, the severity of the affliction, and the concentration of benzoyl peroxide compound as well as the composition of the topical carrier in the particular composition being used.

The examples herein illustrate the present methods and compositions, but are not intended to be limitations thereof.

The following composition illustrates the present invention with the most desired components and formulations.

| Compound | % by Weight |
|---|---|
| Benzoyl Peroxide | 10 |
| Myristyl Alcohol | 2 |
| 1,2-propanediol | 10 |
| Carbopol 940 | 1.35 |
| Carboset 514H | 0.63 |
| NaOH | 0.35 |
| Ethanol | 13.5 |
| Water | Balance |

4.0 grams of B. F. Goodrich Carbopol 940 are dispersed in 200 ml $H_2O$. To this solution is added 50 ml of ethanol. To this mixture is added about 6 pellets (ca. 0.6 g.) of sodium hydroxide. The mixture immediately gels. To a standard, commercially available solution of B.F. Goodrich Carboset 514H (30% active polymer in ammonia water) is added sufficient water to produce a solution containing 5% by weight of the active polymer. 50 ml of this dilute solution is added to the previously prepared gel and the mixture is stirred until a uniform gel is obtained.

To the gel so produced, the other components of the composition are mixed to achieve the desired concentration.

Complete mixing of the components is followed by cold milling using a colloid mill to reduce the particle size of the benzoyl peroxide. The final composition is effective yet reasonably mild to the skin.

The composition thus prepared is then applied as needed to the situs of affliction. Typically, the composition is applied morning and night with fingertips, pads, cotton balls, or the like.

Although 1,2-propanediol is the preferred diol for formulation purposes, 1,2-butanediol 1,3-butanediol, and 2,3-butanediol can also be used in the present compositions to yield enhanced penetration of benzoyl peroxide into keratinous tissue. The following examples are typical of the compositions herein. In the main, the topical application of the compositions of this invention provides excellent anti-acne results over a period of a few days or weeks.

## EXAMPLE I

| Compound | % by Weight |
|---|---|
| Benzoyl Peroxide | 2 |
| Myristyl Alcohol | 3 |
| 1,2-propanediol | 30 |
| Carbopol 940 | 1.35 |
| Carboset 514H | 0.63 |
| NaOH | 0.35 |
| Ethanol | 13.5 |
| Water | Balance |

The Carbopol, Carboset, ethanol, and NaOH are mixed as disclosed above. The remaining ingredients are mixed into the gel which is then cold milled to make an anti-acne preparation which is effective, yet is exceptionally mild to the skin.

EXAMPLE II

| Compound | % by Weight |
|---|---|
| Benzoyl Peroxide | 5 |
| Carboset 514H | 0.63 |
| Ethanol | 13.5 |
| Lauryl alcohol | 5 |
| 1,2-butanediol | 15 |
| Water | Balance |

The compounds are mixed together and cold milled to give an effective anti-acne preparation when applied two to three times daily over a period of one to three weeks. 2,3-Butanediol and 1,3-butanediol can be substituted for 1,2 butanediol to yield similar results. Other $C_6-C_{14}$ primary alcohols, such as n-hexanol, n-heptanol, n-octanol, n-nonanol, n-decanol, and n-undecanol can be substituted for n-lauryl alcohol to achieve similar results.

0013459

- 1 -

CLAIMS

1. A composition, for use in the topical treatment of acne, characterized in that it comprises from about 0.5 to about 25% benzoyl peroxide and from about 8% to about 99% of a penetrating carrier consisting essentially of:

(a) a $C_6$ to $C_{14}$ primary alkanol, or mixtures thereof; and

(b) a diol selected from 1,2-propanediol, 1,2-butanediol, 1,3-butanediol, and 2,3-butanediol, or mixtures thereof, the weight ratio of primary alcohol to diol being in the range from about 5:1 to about 1:20;

the balance of the composition comprising cosmetically acceptable excipients.

2. A composition according to Claim 1 comprising from about 5% to about 30% by weight of the diol.

3. A composition according to Claims 1 or 2 wherein the diol is 1,2-propanediol.

4. A composition according to any one of Claims 1-3 comprising from about 1% to about 40% of the primary alkanol.

5. A composition according to any one of Claims 1-4 wherein the primary alkanol is myristyl alcohol.